(19)

Europäisches
Patentamt
European
Patent Office
Office européen
des brevets

(11) **EP 2 453 452 A1**

(12) **EUROPÄISCHE PATENTANMELDUNG**

(43) Veröffentlichungstag:
**16.05.2012   Patentblatt 2012/20**

(51) Int Cl.:
*H01F 27/40* (2006.01)          *G01N 33/28* (2006.01)
*H01F 27/14* (2006.01)

(21) Anmeldenummer: **10190885.3**

(22) Anmeldetag: **11.11.2010**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**

(71) Anmelder: **Industrie- und Umweltlaboratorium
Vorpommern GmbH
17489 Greifswald (DE)**

(72) Erfinder: **Bräsel, Eckhard
17493 Greifswald (DE)**

(74) Vertreter: **Garrels, Sabine et al
Schnick & Garrels
Patentanwälte
Schonenfahrerstrasse 7
18057 Rostock (DE)**

(54) **Verfahren und Vorrichtung zur Bestimmung des Gasungsverhaltens von flüssigkeitsgefüllten Hochspannungsanlagen mit Gassammelrelais**

(57)      Das Verfahren und die Vorrichtung zur Bestimmung des Gasungsverhaltens von flüssigkeitsgefüllten Hochspannungsanlagen mit Gassammelrelais haben die Aufgabe, das Gasungsverhalten von flüssigkeitsgefüllten Hochspannungsanlagen mit Gassammelrelais auf Basis eines minimalen technischen Aufwandes zuverlässig zu bestimmen, um so eine breite Anwendung zu ermöglichen.

Das erfindungsgemäße Verfahren beginnt mit der Flüssigkeitsfüllung des Separiergefäßes über den Bypass des Steigrohres. Mit der Signalisation der 1. Gasansammlung im Relais wird mittels Überdruck, der sich aus der Höhendifferenz der Flüssigkeitsspiegel zwischen Ausdehnungsgefäß und Gassammelrelais ergibt, ein Teilvolumen Gas über das ansteuerbare Ventil in das Separiergefäß transportiert, solange bis der Schwimmerkontakt im Auffanggefäß dies beendet. Weitere Signalisationen von Gasansammlungen im Relais öffnen das ansteuerbare Ventil solange bis der steigende Ölspiegel im Gassammelrelais einen weiteren Schwimmerkontakt auslöst. Die sich so wiederholenden Gastransporte können durch sich parallel akkumuliertes Gas zeitlich verlängert werden. Der Gastransport führt über das gasgefüllte Separiergefäß in das verbundene Auffanggefäß, das mit der Atmosphäre in Verbindung steht. Eine Steuer- und Auswerteeinheit erfasst die Anzahl der Signalisationen und deren Zeitpunkte.

Figur 2

EP 2 453 452 A1

**Beschreibung**

Gassammelrelais haben in flüssigkeitsgefüllten Hochspannungsanlagen die Aufgabe, ungelöste Gase zu akkumulieren und das Erreichen eines einstellbaren Volumenwertes zu signalisieren (Gasalarm). Das kann in Verbindung mit dem Ansprechen eines Strömungsrelais für spontane Fehler geschehen oder auch für weniger spontane Fehler ohne Ansprechen des Strömungsrelais. Ungelöste Gase entstehen als Folge eines elektrischen oder thermischen Fehlers in der flüssigkeitsgefüllten Hochspannungsanlage oder durch Lufteintrag bzw. -abscheidung. Nur spontane Fehler der Hochspannungsanlage können das Strömungsrelais auslösen, weshalb auch daran die Abschaltung der Hochspannungsanlage gekoppelt ist. Weniger spontane Fehler und Lufteintrag bzw. -abscheidung können nur zum Ansprechen des Gassammelrelais führen. Allgemein wird der Betrieb der Hochspannungsanlage nicht unterbrochen. Steht bei spontanen Fehlern die Ursachenermittlung im Vordergrund, wird diese beim alleinigen Ansprechen des Gassammelrelais durch Risikobewertungen zum Weiterbetrieb bzw. durch Maßnahmen ergänzt. Dazu muss das Gasungsverhalten bestimmt werden, bestehend aus Gasungsrate und Gaszusammensetzung. Da die Risikobewertung anhand der Gasungsrate nicht absolut darstellbar ist, reicht für die Praxis die Bewertung der Gasakkumulationsrate eines Gassammelrelais aus. Wichtige Aussagen betreffen die Zeitverteilung der Rate und ihr Trend. Damit kann in Kauf genommen werden, dass zu möglichen Spitzen freies Gas am Gassammelrelais vorbei in das Ausdehnungsgefäß gelangt.

Die verbleibenden Nachteile des Gassammelrelais sind:

- Freischaltung der Hochspannungsanlage in der Regel, um eine Gasprobe zu entnehmen,
- Standzeit von mehreren Stunden zwischen Signalisation und Probenahme, in welcher sich das Relaisgas verändert, und keine Gasakkumulation erfolgt;
- Fehlen einer zeitaufgelösten Gasakkumulation.

Von besonderer Bedeutung ist, die Gasakkumulation nach dem Signalisationszeitpunkt. Nur so kann man während des Weiterbetriebes der Hochspannungsanlage das tatsächliche Gasungsverhalten darstellen. Vorzugweise mit der unverfälschten Probenahme des Relaisgases zur Analyse sollte die Gasakkumulationsrate bestimmt werden, um das Ergebnis in die Gesamtbewertung nach Vorlage des Analysenergebnisses einbeziehen zu können. Da aber das Vorliegen von Ergebnissen der Gasin-Öl-Analyse, die unbedingt einzubeziehen sind, oft länger dauert, kann der Zeitraum für die Gasakkumulation auch noch ausgedehnt werden.

Nur eine Lösung ist bekannt, die Bestimmung der Gasakkumulationsrate nach der Signalisation des Gassammelrelais durchzuführen und die ist verbunden mit der Gasprobenahme. In WO 2003/098763 A1 ist ein Buchholz-Relais beschrieben, das drei gleichgroße Gasdomeinheiten analog dem herkömmlichen Relais mit jeweils kompletten Schwimmerkontakten besitzt. Jede Einheit signalisiert getrennt und aus den Zeitdifferenzen kann eine Steuer- und Auswerteeinheit die Gasakkumulationsraten ermitteln. Bei Gasfüllung aller drei Einheiten erfolgt über ein steuerbares Ventil am Kopf des Buchholz-Relais der Gastransport hinab in eine Gasprobenahmeeinheit, deren Innendruck über eine Verbindungsleitung zu einem Standrohr, das mit der Öffnung in den Ölraum des Ausdehners führt, mindestens auf Atmosphärendruck gehalten wird. Nach der Gasentleerung ist das Buchholz-Relais weiter einsatzbereit. Als Nachteile dieser Lösung müssen genannt werden:

- Die Bestimmung der Gasakkumulationsraten ist nur mit dem Vielfachen eines einzelnen Gasdomvolumens (herkömmliches Buchholz-Relais) möglich.
- Die Standzeiten bis zur Gasentleerung sind größer als herkömmlich, wodurch die Gasverfälschung zunimmt.
- Durch die Verbindung zu dem Standrohr im Ausdehner ist der Transportdruck bei der Gasentleerung des Buchholz-Relais reduziert, was insbesondere bei flachen Ausdehnungsgefäßen den Einsatz ausschließt.
- Ein Wetterschutz ist für das ansteuerbare Ventil am Buchholz-Relais zusätzlich nötig.
- Beim gasgefüllten Buchholz-Relais bzw. während der Entleerung wird weiteres freies Gas nicht erfasst.
- Der Geräte- und Installationsaufwand insbesondere durch die Einbeziehung des Ausdehners ist sehr groß, eine Nachrüstung von herkömmlichen Buchholz-Relais an arbeitenden Transformatoren ist nicht möglich.

Bekannte Vorrichtungen zur Bestimmung der Gasakkumulationsrate bis zur Signalisation (EP 1 566 874 B1, DE 101 33 615 C1) sind nicht erweiterbar auf die Zeit nach der Signalisation. Sie lassen sich auch nicht mit einer Gasableitungsvorrichtung nach DE 100 63 408 A1 kombinieren, da diese nur einmalig Gas übernehmen kann.

**Darstellung der Erfindung**

Aufgabe der Erfindung ist es, das Gasungsverhalten von flüssigkeitsgefüllten Hochspannungsanlagen mit Gassammelrelais auf Basis eines minimalen technischen Aufwandes zuverlässig zu bestimmen, um so eine breite Anwendung zu ermöglichen. Weiterhin sollen alle Nachteile der bisherigen Lösungen beseitigt werden. Dabei werden die laufende Ermittlung der Gasakkumulationsrate ab der 1. Signalisation des Gassammelrelais sowie die einmalige manuelle Bestimmung der Zusammensetzung des mit der 1. Signalisation von der Flüssigkeit getrennten Relaisgases als ausreichend für die Darstellung des Gasungsverhaltens angesehen.

Diese Aufgabe wird mit Hilfe eines Verfahrens nach den Ansprüchen 1 - 3 und einer Vorrichtung nach den Ansprüchen 4 - 9 gelöst.

Nach der Erfindung wird eine Vorrichtung am Fuße der flüssigkeitsgefüllten Hochspannungsanlage angebracht, diese besteht aus einem miteinander verbundenen Separier- und Auffanggefäß in vertikaler Anordnung. Das Separiergefäß ist zweiteilig zusammengesetzt, der obere Teil besitzt den größeren Durchmesser und am Kopf ein Ventilelement zur Entnahme von Gasproben. Der untere Teil hat einen kleineren Durchmesser und in dessen Boden führt U-förmig eine Steigleitung, die mit dem Entlüftungshahn des Gassammelrelais in Verbindung steht und die ein mit dem Signalkreis des Gassammelrelais verbundenes ansteuerbares Ventil enthält, das bei Signalisation des Gassammelrelais öffnet und eine Flüssigkeitsverdrängung in das Auffanggefäß gestattet. Ein im Auffanggefäß installierter Schwimmerkontakt kann bei Erreichung eines vorgebbaren Flüssigkeitsspiegels das ansteuerbare Ventil schließen. Im Bypass zum ansteuerbaren Ventil befindet sich ein manuell bedienbares Ventil zum Füllen und Entlüften des Separiergefäßes. Das Auffanggefäß besitzt einen Ablaufhahn und am Kopf einen Be- und Entlüftungsstutzen sowie ein skaliertes Schauglas zur Bestimmung kleinerer Gasvolumina. Eine Steuer- und Auswerteeinheit ermöglicht über die Signalkontakte des Gassammelrelais Wiederholungen des Öffnens und Schließens des ansteuerbaren Ventils in der Steigleitung sowie die Bestimmung der Gasakkumulationsrate aus den gespeicherten Daten.

Das erfindungsgemäße Verfahren beginnt mit der Flüssigkeitsfüllung des Separiergefäßes über den Bypass des Steigrohres. Mit der Signalisation der 1. Gasansammlung im Relais wird mittels Überdruck, der sich aus der Höhendifferenz der Flüssigkeitsspiegel zwischen Ausdehnungsgefäß und Gassammelrelais ergibt, ein Teilvolumen Gas über das ansteuerbare Ventil in das Separiergefäß transportiert, solange bis der Schwimmerkontakt im Auffanggefäß dies beendet. Weitere Signalisationen von Gasansammlungen im Relais öffnen das ansteuerbare Ventil solange bis der steigende Ölspiegel im Gassammelrelais einen weiteren Schwimmerkontakt auslöst. Die sich so wiederholenden Gastransporte können durch sich parallel akkumuliertes Gas zeitlich verlängert werden. Der Gastransport führt über das gasgefüllte Separiergefäß in das verbundene Auffanggefäß, das mit der Atmosphäre in Verbindung steht. Eine Steuer- und Auswerteeinheit erfasst die Anzahl der Signalisationen und deren Zeitpunkte.

**Kurze Beschreibung der Abbildungen**

Im Folgenden wird eine Ausführungsform der Vorrichtung und das Verfahren für einen Öltransformator mit Buchholz-Relais, und hier nur die Funktion des Gassammelrelais, beschrieben.

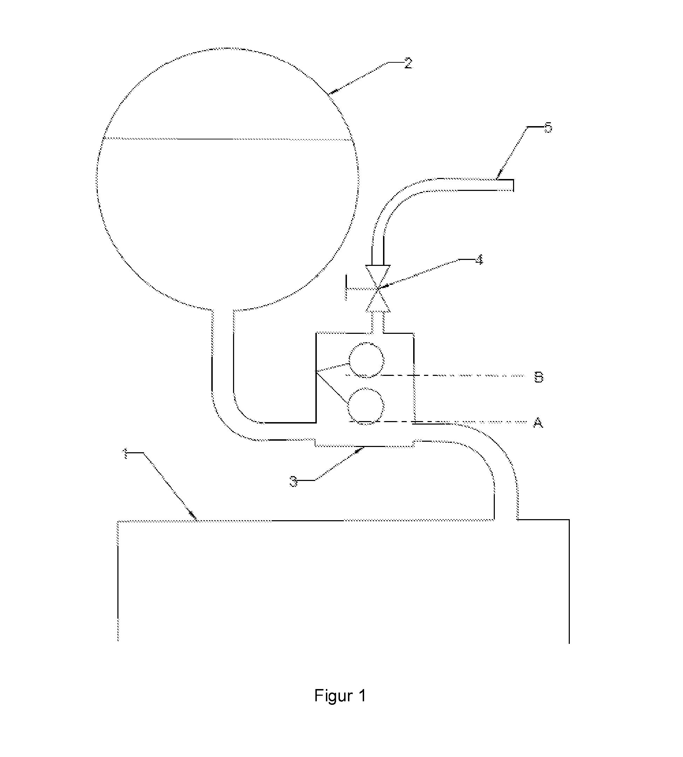

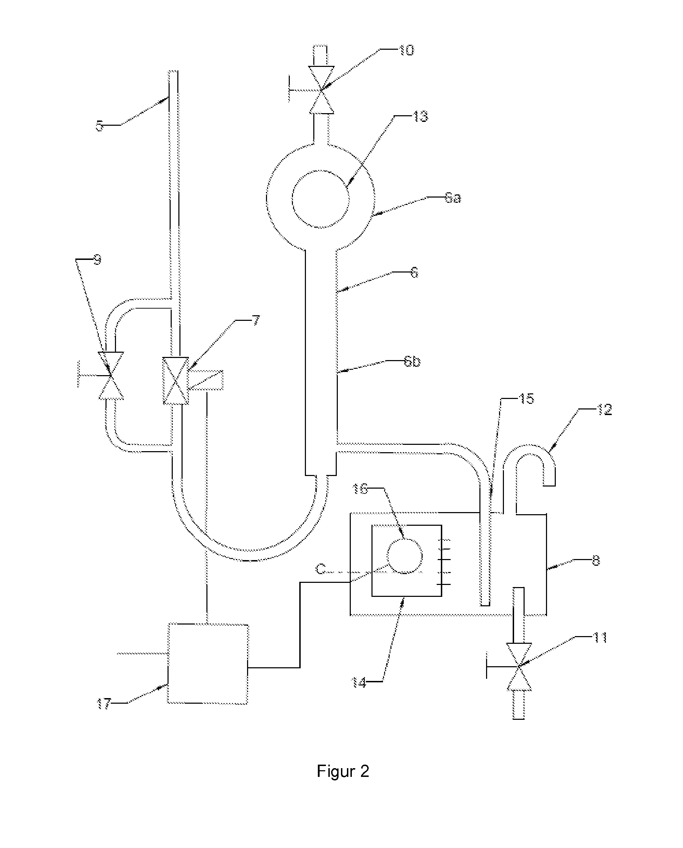

Fig. 1 zeigt eine schematische Darstellung des Öl-/Gassystems eines Transformatormotors mit zwei frei wählbaren Schwimmerkontakten im Buchholz-Relais, die für die Ein- und Ausschaltung der Signalisation genutzt werden. Beim Buchholz-Relais kann das Ein- und Ausschalten durch eine Magnetschaltröhre mit nur einem Schwimmer realisiert werden. Fig. 2 zeigt den schematischen Aufbau einer Ausführungsform der erfindungsgemäßen Vorrichtung.

**Ausführung der Erfindung**

Die beschriebene Ausführungsform der Vorrichtung (Fig. 2) ist am Fuß des Transformators montiert und weist eine absperrbare, zweiteilige Gefäßanordnung mit einem Separiergefäß 6 und einem Ausgleichgefäß 8 auf. Seitlich oberhalb des Bodens des Separiergefäßes 6 ist ein Ablaufrohr 15 angebracht, das vertikal bis zum Boden des Ausgleichsgefäßes 8 führt. Das Separiergefäß 6 besteht aus einem waagerechten Zylinder 6a größeren Durchmessers oben, in dessen Mantel ein vertikaler Zylinder 6b kleineren Durchmessers unten eingesetzt ist. Das Separiergefäß 6 steht über eine Zufuhrleitung 5, die U-förmig unten in den Boden führt und ein an den Signalkreis des Buchholz-Relais ankuppelbares und elektrisch betriebenes Ventilelement 7 besitzt, mit dem Entlüftungshahn 4 des Buchholz-Relais 3 in Verbindung, das in der Verbindungsleitung zwischen Transformatorkessel 1 und Ausdehnungsgefäß 3 eingebaut ist (Fig. 1). In die Zuführleitung 5 ist ein manuell betriebenes Ventilelement 9 als Bypass zum Ventilelement 7 eingebaut. Ein solches Ventilelement 10 befindet sich auch am Kopf des Separiergefäßes 6, was zur Entlüftung und Probenahme dient. Der größere Zylinder 6a des Separiergefäßes 6 besitzt ein Schauglas 13. Ein skaliertes Schauglas 14 besitzt das Ausgleichsgefäß 8, das im Boden ein manuell bedienbares Ventilelement 11 enthält, von dem nach innen ein Rohrstutzen führt und in dessen Kopf ein Ent- und Belüftungsrohr 12 eingesetzt ist, das auch zu einem Sicherheitsventil führen kann.

Im Ausgleichsgefäß 8 befindet sich ein Schwimmer 16. Eine Steuer- und Auswerteeinheit 17 sichert die Stromversorgung von Ventilelement 7 und besitzt einen Anschluss an die Schwimmerkontakte A und B des Buchholz-Relais 3 sowie an den Schwimmerkontakt C des Ausgleichsgefäßes 8.

Im Folgenden werden die einzelnen Verfahrensschritte

- Füllung

- Gasakkumulationszyklen
- Gasentnahme
- Entlüftung

beschrieben.

**Füllung**

Das Buchholz-Relais 3 (Fig. 1) ist vollständig mit Öl gefüllt, der Schwimmer befindet sich in der oberen Endlage. Die Vorrichtung (Fig. 2) ist vollständig leer und über die Zufuhrleitung 5 an den geöffneten Entlüftungshahn 4 des Buchholz-Relais 3 angeschlossen. Alle Ventile 7, 9, 10, 11 sind geschlossen. Das ist der Ausgangszustand nach jeder Installation der Vorrichtung. Zum Füllen werden die Ventile 9, 10 und 11 geöffnet.

Beginnt der Ölablauf über Ventil 11, wird dieses verschlossen. Nun wird das Ent- und Belüftungsrohr 12 verschlossen, vorzugsweise mit dem Daumen. Einige Zeit später wird der Ölspiegel im Schauglas 13 des oberen Zylinders 6a des Separiergefäßes 6 sichtbar. Beginnt der Ölablauf über Ventil 10, wird dieses verschlossen. Der Ent- und Belüftungsrohr 12 kann freigegeben werden und Ventil 9 wird geschlossen. Damit ist der Betriebszustand der Vorrichtung hergestellt (Schauglas 13 ölgefüllt, Schauglas 14 Ölstand an der unteren Markierung).

**Gasakkumulationszyklen**

Bei Gasansammlung sinkt der Ölspiegel im Buchholz-Relais 3 (Fig. 1). Hat der Schwimmer die Kontaktlage A erreicht, erfolgt über eine Magnetschaltröhre die Erzeugung eines Signals. Dieses wird über die Steuer- und Auswerteeinheit 17 zum Ventil 7 übertragen und schließt dort den Stromkreis, womit das Ventil 7 öffnet. Durch den statischen Druck der Ölsäule zwischen den Ölspiegeln im Ausdehner 2 und dem der Kontaktlage A im Buchholz-Relais 3 wird das akkumulierte Gas in die Zufuhrleitung 5 zur Vorrichtung (Fig. 2) gedrückt. Infolge entleert sich die Zufuhrleitung 5 und Gas beginnt in das Separiergefäß 6 einzutreten. Das ist der Zeitpunkt, wo der Schwimmer frühestens die Kontaktlage B erreicht und die Magnetschaltröhre ein Signal für die Steuer- und Auswerteeinheit 17 erzeugt, das beim ersten Mal nicht auf den Stromkreis übertragen wird.

Das Öl fließt aus dem Separiergefäß 6 in das Ausgleichsgefäß 8. Durch den Schwimmerkontakt C wird bei vorgegebener Ölspiegelhöhe ein Signal für die Steuer- und Auswerteeinheit 17 erzeugt, das Ventil 7 schließt. Weitere Signale des Schwimmerkontaktes C werden von der Steuer- und Auswerteeinheit 17 nicht verarbeitet.

Die Abmessungen der Gefäße sind so gewählt, dass zu diesem Zeitpunkt der Ölspiegel im Separiergefäß 6 im unteren Zylinder 6b steht und der Ölspiegel im Ausgleichsgefäß 8 etwa in der Mitte des Schauglases 14 sichtbar ist (Referenzmarke).

In diesem Schaltzustand der Vorrichtung kann das im Separiergefäß 6 eingeschlossene Relaisgas viele Stunden ohne praktische Änderung der Zusammensetzung bis zur Entnahme aufbewahrt bleiben.

Erfolgte im Buchholz-Relais eine "Auslösung" infolge eines spontanen Fehlers, ist der Transformator abgeschaltet und es interessiert nur die Gaszusammensetzung. Dazu ist die Gasprobenahme durchzuführen.

Erfolgte dagegen im Buchholz-Relais eine "Warnung" infolge eines schleichenden Fehlers bzw. einer Luftabscheidung bleibt der Transformator in der Regel in Betrieb. Neben der Probenahme zur Gasanalyse ist die Gasakkumulationsrate zu bestimmen. Das ist frühestens zum Probenahmezeitpunkt interessant und ist abhängig von der Gasungsaktivität des Transformators:

- Feinbestimmung ohne weiteren Gasalarm in Verbindung mit der Entlüftung.
- Grobbestimmung nach weiteren Gasalarmen mit anschließender Entlüftung.

Die notwendigen Angaben hat die Steuer- und Auswerteeinheit 17 gespeichert.

**Gasakkumulationsratenbestimmung (fein)**

Ausgangspunkt ist die beendete Gasentnahme. Ein Schlauch mit Mundstück als Zubehör zur Vorrichtung wird auf das Ent- und Belüftungsrohr 12 gesteckt und vorsichtig gepustet, dabei wird das Ventil 10 solange geöffnet bis Öl abläuft unter Beobachtung des Ölspiegels im Schauglas 13, dann ist das Pusten beendet. Das ist der Ausgangszustand für die volumetrische Bestimmung des Restgases im Buchholz-Relais. Nun wird Ventil 9 geöffnet und der Ölspiegel im Separiergefäß 6 beobachtet. Bei Abfall bis zur Unterkante des Schauglases 13 wird Ventil 9 geschlossen. Alle Gefäßabmessungen sind so gewählt, dass jetzt das gesamte Gasvolumen der 1. Alarmsignalisation erfasst ist. Nun erfolgt ein weiteres Herauspusten des Gases über Ventil 10. Danach wird der Ölhöhenstand ($H_0$) im Ausgleichsgefäß 8 registriert und erneut Ventil 9 geöffnet. Im Schauglas 13 wird der Ölspiegel beobachtet. Hat sich nach der Signalisation weiteres Gas im Buchholz-Relais angesammelt, wird dies jetzt sichtbar. Sinkt der Ölspiegel nicht mehr, wird Ventil 9 geschlossen und der Höhenstand ($H_1$) im Schauglas 14 abgelesen. Anderenfalls wird bei Erreichen der Unterkante des Schauglases 13 das Ventil 9 geschlossen und der Höhenstand ($H_1$) registriert. Das Herauspusten und das Öffnen von Ventil 9 muss bei maximaler Gasansammlung noch einmal wiederholt werden:

Gasakkumulationsrate (fein) =

$$\frac{(H_1 - H_0) + (H_2 - H_0)}{\mathrm{Pr\,obenahmezeitpunkt - Alarmzeitpunkt}} \left[\frac{cm^3\,Gas}{h}\right]$$

Nach der letzten Gasansammlung im Separiergefäß 6 erfolgt die Entlüftung.

**Gasakkumulationsratenbestimmung (grob)**

Treten weitere Gasalarme auf, erreicht jedes Mal der Schwimmer die Kontaktlage A im Buchholz-Relais 3 und die Magnetschaltröhre sendet ein Signal an die Steuer- und Auswerteeinheit 17, die das Ventil 7 öffnet. Im Unterschied zum 1. Gasalarm wird mit Erreichen der Kontaktlage B im Buchholz-Relais 3 jetzt jedes Mal durch die Steuer- und Auswerteeinheit 17 das Ventil 7 geschlossen. In Steuer- und Auswerteeinheit 17 sind alle Schwimmerkontakt A/B - Signale mit der Uhrzeit ($t_A$, $t_B$) gespeichert. Sie können als Zeitverlauf dargestellt sowie weiter berechnet werden als:

Gasakkumulationsrate (grob) =

$$\frac{V_R + \left(\dfrac{\sum}{n}\, t_B - t_A\right) A_0 + < V_{B-A}}{\mathrm{Pr\,obenahmezeitpunkt - 1.\,Alarmzeitpunkt}} \left[\frac{cm^3\,Gas}{h}\right]$$

wobei

$$A_0 = \frac{V_{B-A}}{(t_B - t_A)\,min}$$

die Transportrate ist.

$V_R$ = Referenzmarken - Volumen

$V_{B-A}$ = Differenzvolumen der Schwimmermarken

n = Anzahl der Schaltungen von Ventil 7 durch den Schwimmerkontakt B

Nach dem letzten Gasalarm erfolgt die Entlüftung.

Die Gasakkumulationsratenbestimmungen können auch zu einem späteren Zeitpunkt nach den Gasprobenahmen durchgeführt werden.

**Gasentnahme**

Die Entnahmetechnik wird an Ventil 10 angeschlossen und nach Öffnen des Ventils 10 erfolgt bei leichtem Unterdruck die Probenahme. Dabei wird im Schauglas 13 der Ölspiegel sichtbar. Ventil 10 wird dann rechtzeitig wieder geschlossen. Nach jeder Gasprobenahme sollte das Buchholz-Relais vollständig entlüftet werden.

**Entlüftung**

Dazu werden Ventil 9 und 10 geöffnet. Im Schauglas 13 wird das Austreten von Gasblasen sichtbar. Ist dies beendet, tritt anschließend Öl in Ventil 10 aus, das dann zu schließen ist. Nun wird auch Ventil 9 geschlossen. Ventil 11 wird solange geöffnet bis kein Öl mehr abläuft. Mit dem Drücken der "Reset-Taste" an der Steuer- und Auswerteeinheit 17 ist die Vorrichtung wieder einsatzbereit.

Wird der Bestimmung der Gasakkumulationsrate weniger Bedeutung beigemessen, kann der Schwimmerkontakt C anstatt aus dem Ausgleichsgefäß 8 durch einen weiteren, hier weiter nicht gezeigten Schwimmerkontakt D aus dem Buchholz-Relais ersetzt werden, um das Ventil 7 in der Zuführleitung 5 zu schließen..

Die Vorrichtung ist am Fuße des Transformators befestigt und besitzt eine vorgegebene Länge der Rohrleitung (5).

Die erfindungsgemäße Vorrichtung kann in einem Gehäuse untergebracht sein und eine Erhaltungsheizung besitzen.

**Patentansprüche**

1. Verfahren zur Bestimmung des Gasungsverhaltens von flüssigkeitsgefüllten Hochspannungsanlagen mit Gassammelrelais auf Basis von Gasakkumulationsrate und -zusammensetzung unter Verwendung einer Vorrichtung, bestehend aus einem externen Separiergefäß (6) und einem Ausgleichsgefäß (8), **dadurch gekennzeichnet,**

   - **dass** die Vorrichtung, welche mit dem Gassammelrelais verbunden ist, über eine Zuführleitung (5) mit Öl gefüllt wird,
   - **dass** bei Gasansammlung im Buchholz-Relais (3) ein unterer Schwimmerkontakt (A) des Gassammelrelais geschaltet wird wodurch ein Ventil (7) öffnet, und durch den hydrostatischen Druck ein Teilvolumen Relaisgas zur unverfälschten Aufbewahrung bis zur Probenahme für die Gasanalyse über die Zuführleitung (5) in das Separiergefäß (6) transportiert wird, welches mit dem Ausgleichsgefäß (8) verbunden ist, in dem ein Schwimmerkontakt (C) den Transport beendet, indem das Ventil (7) in der Zuführleitung (5) geschlossen wird,
   - wonach im Falle des Weiterbetriebes der Anlage die volumetrische Bestimmung der Gasakkumulationsrate zwischen dem Zeitpunkt des Ansprechens des unteren Schwimmerkontaktes (A) des Gassammelrelais und der Probenahme für den Fall, dass der untere Schwimmerkontakt (A) kein weiteres mal angesprochen wird, erfolgt,
   - oder im Falle des Ansprechens jedes weiteren unteren Schwimmerkontaktes (A) des Gassammelrelais in Verbindung mit dem Ansprechen eines oberen Schwimmerkontaktes (B) des Gassammelrelais, welche zeitnah erfasst werden, weitere Teilvolumina Relaisgas über das nunmehr gasgefüllte Separiergefäß (6) in das zur Atmosphäre offene Ausgleichsgefäß (8) transportiert werden und
   - aus den registrierten Zeiten sowohl eine zeitliche Verteilung als auch eine akkumulierte Zeit für eine Berechnung der Gasakkumulationsrate mittels einer Transportrate erfolgt.

2. Verfahren nach Anspruch 1 **dadurch gekennzeichnet, dass** die Transportrate der Gasüberführung sich aus dem Quotienten der Volumendifferenz sowie der kleinsten Zeitdifferenz zwischen unterem (A) und oberem Schwimmerkontakt (B) ergibt.

3. Verfahren nach Anspruch 1 **dadurch gekennzeichnet, dass** das Teilvolumen Relaisgas zur unverfälschten Aufbewahrung bis zur Probenahme für die Gasanalyse über die Zuführleitung (5) in das Separiergefäß (6) transportiert wird und ein dritter Schwimmerkontakt (D) im Buchholz-Relais (3) den Transport beendet.

4. Vorrichtung zur Bestimmung des Gasungsverhaltens von flüssigkeitsgefüllten Hochspannungsanlagen mit Gassammelrelais auf Basis von Gasakkumulationsrate und -zusammensetzung, die aus einem mit einem Ausgleichsgefäß (8) verbundenem Separiergefäß (6) besteht, **dadurch gekennzeichnet,**

   - **dass** das Separiergefäß (6) über eine Zuführleitung (5), die ein ansteuerbares Ventil (7) mit einem Bypaß mit Ventil (9) besitzt, flüssigkeitsseitig über einen Entlüftungshahn (4) mit dem Gassammelrelais (3) verbunden ist,
   - **dass** das Separiergefäß (6) weiterhin über ein seitliches Ablaufrohr (15) in ein Ausgleichsgefäß (8) führt, welches ein in den Boden hineinragendes Rohr mit Ventil (11), im oberen Bereich ein Ent- und Belüftungsrohr (12) sowie ein skaliertes Schauglas (14) und einen Schwimmer (16) mit Schwimmerkontakt (C) besitzt, und
   - **dass** eine Steuer- und Auswerteeinheit (17), welche mit dem ansteuerbaren Ventil (7) und dem Schwimmerkontakt (C) verbunden ist, über eine Signalleitung eine Verbindung zu den beiden Schwimmerkontakten (A) und (B) des Gassammelrelais (3) besitzt.

5. Vorrichtung nach Anspruch 4, **dadurch gekennzeichnet, dass** das Separiergefäß (6) aus einem waagerechten Zylinder (6a) größeren Durchmessers besteht, der ein Schauglas (13) und einen Entlüftungshahn (10) besitzt, und einem vertikalen Zylinder (6b) kleineren Durchmessers zusammengefügt ist, dessen Boden mit der Zuführleitung (5) verbunden ist.

6. Vorrichtung nach Anspruch 4, **dadurch gekennzeichnet, dass** diese am Fuße des Transformators befestigt ist und eine vorgegebene Länge der Zuführleitung (5) besitzt.

7. Vorrichtung nach Anspruch 4, **dadurch gekennzeichnet, dass** diese in einem Gehäuse untergebracht ist, das eine Erhaltungsheizung besitzen kann.

8. Vorrichtung nach Anspruch 4, **dadurch gekennzeichnet, dass** das Ent- und Belüftungsrohr (12) mit einem Sicherheitsventil verbunden ist.

9. Vorrichtung nach Anspruch 4, **dadurch gekennzeichnet, dass** ein dritter Schwimmerkontakt (D) im Buchholz-Relais (3) in der Art angeordnet ist, um das Ventil (7) in der Zuführleitung (5) zu schließen.

Figur 1

Figur 2

**Europäisches Patentamt**
**European Patent Office**
**Office européen des brevets**

## EUROPÄISCHER RECHERCHENBERICHT

Nummer der Anmeldung

EP 10 19 0885

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,D | DE 100 63 408 A1 (BRAESEL ECKHARD [DE]) 4. Juli 2002 (2002-07-04) * Spalte 2, Absatz 10 - Spalte 3, Absatz 13; Abbildungen 1,2 * ----- | 1-9 | INV. H01F27/40 G01N33/28 H01F27/14 |
| A,D | WO 03/098763 A1 (WILLIAMS JOHN OWEN [AU]) 27. November 2003 (2003-11-27) * Zusammenfassung; Abbildungen 1,2 * ----- | 1-9 | |

**RECHERCHIERTE SACHGEBIETE (IPC)**

H01F
G01N

Der vorliegende Recherchenbericht wurde für alle Patentansprüche erstellt

| Recherchenort | Abschlußdatum der Recherche | Prüfer |
|---|---|---|
| München | 5. April 2011 | Kardinal, Ingrid |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

.........................................................................

& : Mitglied der gleichen Patentfamilie, übereinstimmendes Dokument

EPO FORM 1503 03.82 (P04C03)

EP 2 453 452 A1

**ANHANG ZUM EUROPÄISCHEN RECHERCHENBERICHT
ÜBER DIE EUROPÄISCHE PATENTANMELDUNG NR.**

EP 10 19 0885

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-04-2011

| Im Recherchenbericht angeführtes Patentdokument | Datum der Veröffentlichung | Mitglied(er) der Patentfamilie | Datum der Veröffentlichung |
|---|---|---|---|
| DE 10063408 A1 | 04-07-2002 | KEINE | |
| WO 03098763 A1 | 27-11-2003 | AU 2002100385 A4 | 20-06-2002 |

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2003098763 A1
- EP 1566874 B1
- DE 10133615 C1
- DE 10063408 A1